(19) Europäisches Patentamt / European Patent Office / Office européen des brevets

(11) **EP 2 564 860 B1**

(12) **EUROPEAN PATENT SPECIFICATION**

(45) Date of publication and mention of the grant of the patent:
**04.03.2015 Bulletin 2015/10**

(21) Application number: **11775278.2**

(22) Date of filing: **27.04.2011**

(51) Int Cl.:
*A61K 36/064* (2006.01)     *A61P 3/04* (2006.01)
*A61P 3/06* (2006.01)       *A61P 3/10* (2006.01)
*A61P 39/06* (2006.01)      *A61P 9/10* (2006.01)

(86) International application number:
**PCT/KR2011/003116**

(87) International publication number:
**WO 2011/136573 (03.11.2011 Gazette 2011/44)**

(54) **YEAST HYDROLYSATE HAVING OBESITY TREATMENT EFFECTS AND ANTIOXIDANT ACTIVITY**

HEFEHYDROLYSAT MIT ANTIOXIDATIVER WIRKUNG ZUR BEHANDLUNG VON ADIPOSITAS

HYDROLYSAT DE LEVURE AYANT DES EFFETS DE TRAITEMENT DE L'OBÉSITÉ ET UNE ACTIVITÉ ANTIOXYDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2010 KR 20100039717**

(43) Date of publication of application:
**06.03.2013 Bulletin 2013/10**

(73) Proprietor: **Neo Cremar Co., Ltd.**
**Seongnam-si, Gyeonggi-do 462-737 (KR)**

(72) Inventors:
• **KIM, Jae-Hwan**
**Seoul 138-202 (KR)**
• **SUH, Hyung-Joo**
**Seoul 158-050 (KR)**

(74) Representative: **Vossius & Partner**
**Siebertstrasse 3**
**81675 München (DE)**

(56) References cited:
**KR-A- 20040 097 813     KR-A- 20050 112 777**

• **JUNG EUN YOUNG ET AL: "Effects of yeast hydrolysate on neuropeptide Y (NPY) and tryptophan hydroxylase (TPH) immunoreactivity in rats", PHYTOTHERAPY RESEARCH, JOHN WILEY & SONS LTD, GB, vol. 23, no. 5, 1 May 2009 (2009-05-01), pages 619-623, XP009174323, ISSN: 1099-1573**
• **DATABASE WPI Week 201038 Thomson Scientific, London, GB; AN 2010-G60745 XP002716943, & KR 2005 0116850 A (UNIV FOUND DAEGU) 13 December 2005 (2005-12-13)**
• **CHOI Y M ET AL: "Characteristics of fermentation and bioavailability of isoflavones in Korean soybean paste (doenjang) with application of Bacillus sp. KH-15", INTERNATIONAL JOURNAL OF FOOD SCIENCE AND TECHNOLOGY, BLACKWELL SCIENTIFIC PUBLICATIONS, OXFORD, GB, vol. 42, no. 12, 1 December 2007 (2007-12-01), pages 1497-1503, XP009174324, ISSN: 0950-5423 [retrieved on 2007-12-07]**
• **KIM K M; ET AL: 'Yeast hydrolysate reduces body fat of dietary obese rats' PHYTOTHERAPY RESEARCH vol. 18, January 2004, pages 950 - 953, XP003014251**
• **VINCENZO BRANDOLINI, ET AL.: 'Influence of Saccharomyces cerevisiae strains on wine total antioxidant capacity evaluated by photochemiluminescence' WORLD JOURNAL OF MICROBIOLOGY AND BIOTECHNOLOGY vol. 23, no. 4, 2007, pages 581 - 586, XP019485252**

EP 2 564 860 B1

• I H KIM, K KIM, AND S G RHEE: 'Induction of an antioxidant protein of Saccharomyces cerevisiae by O2, Fe3+, or 2-mercaptoethanol' PROC. NATL. ACAD. SCI. vol. 86, no. 16, 1989, pages 6018 - 6022, XP055070214

• EUN YOUNG JUNG, ET AL.: 'WEIGHT REDUCTION EFFECTS OF YEAST HYDROLYSATE BELOW 10 kDa ON OBESE YOUNG WOMEN' JOURNAL OF FOOD BIOCHEMISTRY vol. 35, no. 2, April 2011, pages 337 - 350, XP055070216

Description

[Technical Field]

The present invention disclosed herein relates to a yeast hydrolysate which inhibits fat deposition in the body and has antioxidant activity.

[Background Art]

Obesity is a condition in which a body transforms excess calories, that is, the remaining calories after the expenditure of ingested calories, into fat and accumulates the fat around various parts of the body, especially at subcutaneous tissues and in the abdominal cavity. Neutral fat (TG, triglyceride) is an ester-linked compound of glycerin and three fatty acid molecules, and since animal adipose tissues store neutral fats synthesized from carbohydrates, free fatty acids are released into blood by enzymatic hydrolysis of neutral fats. When carbohydrate is insufficient as an energy source, neutral fats stored in the adipose tissues are decomposed into nonesterified fatty acids (NEFA) and glycerol and released into blood. The remaining nonesterified fatty acids after expenditure as an energy source are transformed again into neutral fats in a liver. When these neutral fats are flowed into blood again, these are called as endogenous neutral fats. Excessive neutral fats are closely related with atherosclerosis, coronary artery diseases, and the like.

Obese patient is one whose fat accumulated in the body is much more than required for the function of adipose tissues, and thus, there is a disability in normal biochemical and physiological functions of human body. Obesity is not only a cause of various diseases, such as diabetes, hyperlipidemia, hypertension and coronary artery diseases, and joint diseases, but also makes it impossible for obese patients to live a normal social life.

In addition, obesity is risky to health of animals, especially pets, as well as humans. Pets, especially dogs, cats, and hamsters often get obese or experience nutritional imbalance due to excessive feed intake or imbalanced nutrition intake.

Nutritive components required for animals depend on mainly feeds, and lipids contained in the feeds undergo deterioration in quality due to rancidity during processing and storage and become to show unpleasant tastes and smells. In addition, oxidation products can result in DNA damage or inactivate enzymes in the body to cause metabolic disorders or various diseases. In order to inhibit lipid oxidation, antioxidants such as BHT, BHA, TBHQ, and the like are added. However, those antioxidants exhibit excellent antioxidant effects, but they have problems of mutagenicity and carcinogenicity and due to consumer rejection, the use of such antioxidants has been decreased. Accordingly, many researches on natural antioxidants which have excellent antioxidant activities and secured safety are needed; however, development and industrialization of natural antioxidants are so difficult that many food industries use mainly synthetic antioxidants.

Thus, the present inventors have studied methods for treating or preventing obesity by controlling appetite and the body's metabolism, and found that when yeast was hydrolyzed by a specific enzyme, the yeast hydrolysate could inhibit fat deposition and degrade body fat, and thus have excellent effects on obesity treatment and prevention, thereby leading to completion of the present invention.

[Disclosure]

[Technical Problem]

The present invention provides a composition for use treating and preventing obesity.
The present invention also provides a composition having antioxidant activity.

[Technical Solution]

The present invention provides a yeast hydrolysate obtained by hydrolyzing yeast with a specific enzyme.

[Advantageous Effects]

The yeast hydrolysate of the present invention can reduce body weight and prevent and treat obesity through inhibition of fat deposition in the body and lipolysis. The yeast hydrolysate of the present invention has also antioxidant activity. In addition, the yeast hydrolysate of the present invention prevents and relieves oxidative damage of tissues and cells. Accordingly, the yeast hydrolysate of the present invention has therapeutic and preventive effects on obesity and oxidative damage.

**[Description of Drawings]**

FIG. 1 shows the protein recovery of yeast hydrolysate obtained through hydrolysis using various enzymes.
FIG. 2 shows effects of the yeast hydrolysate on the amounts of glycerol and leptin release in 3T3-L1 adipocytes.
FIG. 3 shows cytotoxicity of the yeast hydrolysate against 3T3-L1 adipocytes, measured by MTT method.
FIG. 4 shows the ability of yeast KH-15 hydrolysate to scavenge DPPH and ABTS radicals in rats.
FIG. 5 shows effects of yeast KH-15 hydrolysate on body weight of SD rats.
FIG. 6 shows the inhibitory effect of yeast KH-15 hydrolysate on fat deposition in SD rats.
FIG. 7 shows effects of yeast KH-15 hydrolysate on body weight of dogs.
FIG. 8 shows effects of yeast KH-15 hydrolysate on abdominal circumference of dogs.
FIG. 9 shows the amounts of blood MDA and GSH in dogs administered yeast KH-15 hydrolysate.

**[Best Mode]**

The present invention is defined by the claims.
In accordance with an embodiment the present invention, a composition comprises a yeast hydrolysate as an effective component and has body weight reducing and antioxidant activities, wherein the yeast hydrolysate is obtained by proteolyzing *Saccharomyces cerevisiae* with a protease KH-15 consisting of the amino acid sequence of SEQ ID NO: 1.
In accordance with another embodiment of the present invention, the composition of the invention is the use in preventing, or treating arteriosclerosis, visceral obesity, abdominal obesity, hyperlipidemia, fatty liver or obesity.
In accordance with an instansice of the present disclosure, a method of preparing a composition having body weight reducing and antioxidant activities includes adding an enzyme to yeast.
The yeast of the present disclosure may be any yeast which is used for foods, and the kind is not particularly limited. The yeast of the present invention is *Saccharomyces cerevisiae any years selected from the group consisting of: Saccharomyces carlsbergensis, Saccharomyces fermentati, Saccharomyces bayanus, Saccharomyces sake, Saccharomyces mandshuricus" Saccharomyces anamensis, Saccharomyces formosensis, Saccharomyces ellipsoideus, Saccharomyces coreanus may be used in the present disclosure.*
The enzyme of the present disclosure may be a protease. Preferably, the protease may be one selected from the group consisting of Protamex, Proleather FG-F, Flavourzyme, Protease A, Aroase AP-10, Pescalase, Papain, KH-15 including the nucleotide sequence of SEQ ID NO:I, Bromelain, Ficin, and Neutrase. More preferably, the protease may be Protamex, Flavourzyme, and KH-15. Most preferably, the protease may be KH-15. However, since a characteristic of the present disclosure comprising the invention is to hydrolyze yeast and thus prepare a yeast hydrolysate which reduces body weight without reducing dietary intake and has antioxidant activity, in one instance of the present disclosure, any protease which hydrolyzes yeast so as to exhibit this characteristic may be include.
The composition may be one selected from a food composition, a feed composition, a pharmaceutical composition, and a cosmetic composition.
The composition of the present invention can remove reactive oxygen species in the body, and thus inhibit fats, cholesterols, and reactive oxygen species in the body from reacting and forming lipid peroxides, and thus prevent the formation of thrombus and embolus. In addition, the composition of the present invention can remove reactive oxygen species, and thus prevent and treat reactive oxygen species-associated thrombus and embolus, lipid peroxides-induced blood circulation disorders, cerebral apoplexy, stroke, cerebral thrombosis, myocardial infarction, arteriosclerosis, and can inhibit reactive oxygen species-induced denaturation of DNAs, cells, and tissues, and thus prevent and treat atopic diseases, allergy, tumors, arthritis, cataract, skin tumor, and inhibit the aging process.
In addition, the composition of the present invention prevents and/or treats a disease selected from the group consisting of arteriosclerosis, visceral obesity, abdominal obesity, hyperlipidemia, fatty liver, and obesity, and prevents diabetes. In addition, the food The composition of the present invention exhibits complex effects on diseases associated with increase in blood lipid concentration, neutral fat accumulation, and the like, in addition to the above diseases. Long-term administration of the food composition, feed composition, or pharmaceutical composition of the present invention exhibits effects of the body weight management, aging retardation through antioxidation, and health improvement.
The present disclosure also provides the food composition including the yeast hydrolysate as an effective component. Examples of the food include, but are not limited to, health supplement foods, health functional foods, functional foods, and the like, and include also natural foods, processed foods, and general food supplies, to which the yeast hydrolysate of the present invention was added.
The food composition including the yeast hydrolysate as an effective component may be added as it is, or may be used along with other foods or food compositions. The food composition including the yeast hydrolysate as an effective component may be used appropriately according to general methods. A mixing amount of the effective component may be determined appropriately depending on its purposes (prevention, health care, or therapeutic treatment). Generally, health functional foods of the present invention may be added in an amount of 0.01 to 70.00 weight%, preferably 0.01 to 30.00 weight%, and more preferably 0.01 to 10.00 weight% with respect to raw materials for preparation of foods or beverages. The effective amount of the yeast hydrolysate in the food composition may be in accordance with the effective amount of the pharmaceutical composition; however, the effective amount of the yeast hydrolysate in the food composition for the long term intake for health and hygiene, or health control may be below the above range. Since the effective component does not have any safety problems, it may be used in a larger amount than the above range.

There is no particular limit to the kind of the food. The food composition including the yeast hydrolysate as an effective component may be used as formulations for oral administration, such as tablets, hard or soft capsules, liquid formulations, suspensions, and the like. These formulations may be prepared using acceptable general carriers, for example, for formulations for oral administration, excipients, binders, disintegrators, lubricants, solubilizers, suspending agents, preservatives, or diluents.

Examples of foods to which the yeast hydrolysate may be added include meat, sausages, breads, chocolates, candies, snacks, confectionery, pizzas, ramen, other noodles, gums, dairy products including ice creams, all sorts of soup, beverages, teas, drinks, alcohol beverages, vitamin complex, and the like, but are not limited to such kinds of foods.

The feed composition including the yeast hydrolysate of the present invention as an effective component may be served along with conventional feeds. The feed composition of the present disclosure may also be added to conventional feed compositions to prepare functional feed compositions. The feed composition of the present disclosure may further include functional components, in addition to the yeast hydrolysate of the present invention. For the preparation of functional feed composition in which the above conventional feed composition and the yeast hydrolysate of the present invention are mixed, the yeast hydrolysate of the present invention may be added in an amount of 0.01 to 30.00 weight%, preferably 0.01 to 20.00 weight%, with respect to the entire feed composition. The effective amount of the yeast hydrolysate in the feed composition may be in accordance with the effective amount of the food composition; however, the effective amount of the yeast hydrolysate in the feed composition for long term intake continuous body weight management or health control may be below the above range. Since the effective component does not have any safety problems, in the case of extreme obesity, it may also be used in a larger amount than the above range.

The feed composition of the present disclosure is for domestic animals or domestic fowls. The domestic animals or domestic fowls are cattle, pigs, chicken, horses, sheep, donkeys, mules, wild boars, rabbits, quails, domestic ducks, roosters, game fowls, doves, turkeys, dogs, cats, monkeys, hamsters, mice, rats, mynahs, parrots, budgies, canaries, and the like, but are not limited to such. Any nonhuman mammals or birds that can be raised domestically may be objects of the feed composition of the present invention.

The composition comprising the yeast hydrolysate of the present invention prevents and treats thrombus, embolus, lipid peroxides-induced blood circulation disorders, cerebral apoplexy, stroke, cerebral thrombosis, myocardial infarction, and arteriosclerosis, and inhibits reactive oxygen species-induced denaturation of DNAs, cells, and tissues, and thus prevents and treats atopic diseases, allergy, tumors, arthritis, cataract, skin tumor, and inhibits the aging process. The composition comprising the yeast hydrolysate of the present invention as an effective component prevents and treats hyperlipidemia, fatty liver, partial obesity such as visceral obesity, abdominal obesity, and the like, and obesity (that is, general obesity).

The composition comprising the yeast hydrolysate of the present invention as an effective component may be administered orally or parenterally, and may be used in forms of medicine and medical formulations. Examples of preferable pharmaceutical formulations are formulations for oral administration such as tablets, hard or soft capsules, liquid formulations, suspensions, and the like. These pharmaceutical formulations may be prepared using pharmaceutically acceptable general carriers, for example, for formulations for oral administration, excipients, binders, disintegrators, lubricants, solubilizers, suspending agents, preservatives, or diluents.

The dose of the pharmaceutical composition comprising the yeast hydrolysate of the present invention as an effective component may be determined by an expert depending on various factors such as condition, age, gender of a patient, complications, and the like. Generally, the pharmaceutical composition including the yeast hydrolysate of the present invention as an effective component may be administered in a dose of from 0.1 mg to 10 g, preferably 10 mg to 1 g, per kg body weight of an adult. A daily dose of the pharmaceutical composition, or 1/2, 1/3, or 1/4 of the daily dose of the pharmaceutical composition may be contained in a unit formulation and the pharmaceutical composition may be administered 1 to 6 times a day. However, the dose for the long term intake for health and hygiene, or health control may be below the above range and since the effective component does not have any safety problems, it may be used in a larger amount than the above range.

The present disclosure also provides the cosmetic composition including the yeast hydrolysate prepared by the preparation method as an effective component, and provides a method for preparing the cosmetic composition including preparing the yeast hydrolysate. In the method for preparing the cosmetic composition, carriers acceptable for cosmetic preparation may be different depending on the formulation of the cosmetic composition of the present diclosure. The cosmetic composition of the present disclosure may be formulated as solutions, suspensions, emulsions, pastes, gels, creams, lotions, soaps, shampoos, surfactant-containing cleansing oils, powered foundations, liquid foundations, cream foundations, sprays, and the like. The antioxidant cosmetic composition of the present invention may be formulated as conventional cosmetics, more particularly skin softners (skin tonics, skin toners), astringents, skin lotions, nutritious creams, massage creams, essences, gels, patches for attaching to skin, powders, ointments for external use, plasters, suspensions, emulsion sprays, eye creams, cleansing creams, cleansing foams, cleansing waters, packs, hair essences, or beauty essences.

When the cosmetic composition prepared by the preparation method of the present disclosure is formulated as pastes, creams, or gels, carriers may be animal oils, vegetable oils, wax, paraffin, starches, trakinds, cellulose derivatives, polyethylene glycols, silicon, bentonite, silica, talc, or zinc oxide.

When the cosmetic composition including the yeast hydrolysate prepared by the preparation method of the present disclosure as an effective component is formulated as solutions or emulsions, carriers may be solvents, solubilizers, or emulsifiers. Examples of such carriers may be water, ethanol, isopropanol, ethyl carbonate, ethyl acetate, benzyl alcohol, benzyl benzoate, propylene glycol, 1,3-butylglycol oil, glycerol aliphatic esters, polyethylene glycols, or fatty acid esters of sorbitan.

When the cosmetic composition including the yeast hydrolysate prepared by the preparation method of the present disclosure is formulated as suspensions, carriers may be liquid diluents such as water, ethanol, or propylene glycol; ethoxylated isostearyl alcohols; aluminum methahydroxides; bentonite; agar; or tragacanth.

When the cosmetic composition prepared by the preparation method of the present disclosure is formulated as surfactant-containing cleansings, carriers may be aliphatic alcohol sulfates, aliphatic alcohol ether sulfates, sulfosuccinic monoesters, isethionates, imidazolinum derivatives, methyltaurate, sarcosinate, fatty acid amide ether sulfates, alkyl amidobetain, aliphatic alcohols, fatty acid glycerides, fatty acid diethanolamides, vegetable oils, lanolin derivatives, ethoxylated glycerol, or fatty acid esters.

**[Mode for Invention]**

Hereinafter, the present invention will be described below in more detail by the following experiments

<Embodiment 1> Materials

Proteases

*Bacillus sp.* KH-15, which was isolated from soybean paste by Department of Food and Nutrition at Korea University and had been kept, was used as the protease-producing strain to obtain a protease, KH-15. That is, a liquid medium in which 1% skim milk was added to LB broth (bactotryptone 10 g/L, yeast extract 5 g/L, NaCl 10 g/L) was used as a medium for producing proteases. The medium for producing proteases was adjusted to pH 6.5, sterilized under pressure at 121°C for 15 min, incubated at 30°C for 24 hrs, and centrifuged to collect the supernatant. The supernatant was used as crude enzyme extract. The nucleotide sequence of the obtained protease KH-15 equates to SEQ ID NO: 1.
<SEQ ID NO: 1>

    MNQKAMIVIA  AGSMLFGGAG  VYAGINLLEM  DKPQTAAVPA

    TAQADSERDK AMDKIEKAYE

LISNEYVEKV DREKLLEGAI QGMLSTLNDP YSVYMDKQTA KGGSDSLDSS FEGIGAEVGM

EDGKIIIVSP FKKSPAEKAG LKPLSTIISI NGESMAGKDL NHAVLKIRGK KGSSVSMKIQ

RPGTKKQLSF RIKRAEIPLE TVFASEKKVQ GHSVGYIAIS TFSEHTAEDF AKALRELEKK

EIEGLVIDVR GNPGGYLQSV EEILKHFVTK DQPYIQIAER NGDKKRYFST LTHKKAYPVN

VITDKGSASA SEILAGALKE AGHYDVVGDT SFGKGTVQQA VPMGDGSNIK LTLYKWLTPN

GNWIHKKGIE PTIAIKQPDY FSAGPLQLKE PLKVDMNNED VKHAQVLLKG LSFDPGREDG

YFSKDMKKAV MAFQDQNKLN KTGVIDTRTA ETLNQQIEKK KSDEKNDLQL QTALKASFVN

Protamex and Flavourzyme were purchased from Novo Korea (Seoul, South Korea); and Proleather FG-F and Protease A were purchased from Amano Enzyme USA Co. (Lombard, IL, USA); and Aroase AP-10 and Pescalase were purchased from Yakult Phannaceutical Ind. Co. (Tokyo, Japan) and DSM Gist (Netherlands), respectively. Their characteristics are as in the following Table 1.

[Table 1]

| Enzyme | source | Optimal condition | | Class |
| --- | --- | --- | --- | --- |
| | | Temp (°C) | pH | |
| Protamex | *Bacillus sp.* | 35-60 | 6.6-7.5 | Complex |
| Flavourzyme | *Aspergillus sp.* | 45-50 | 5.0-7.0 | Complex |
| Proleather | *Bacillus sp.* | 60 | 10 | Endo |
| Protease A | *Aspergillus oryzae* | 50 | 7.0 | Exo |
| Aroase AP-10 | *Bacillus subtilis* | 50-55 | 7.0-8.0 | - |
| Pescalase | *Bacillacs subtilis* | 60 | 8.0 | Endo |
| Papain | *Carica papaya* | 50 | 6.5 | Complex |

Statistical analysis

Data were analyzed using SPSS program and are reported as the mean and standard error. The significant differences among treatment groups were evaluated by Duncan's multiple range test at $p < 0.05$.

<Embodiment 2> Activities of yeast hydrolysate

<Experimental embodiment 1> Preparation of yeast hydrolysate

*Saccharomyces cerevisiae* ATCC4126 was incubated in a liquid medium that contained 2% molasses, 0.6% $(NH_4)_2SO_4$, 0.1 %, $MgSO_4 \cdot 7H_2O$, 0.2 % $KH_2PO_4$, 0.03 % $K_2HPO_4$ and 0.1 % NaCl for 3 days at 30°C. After incubation, the culture was centrifuged at 3,000 rpm for 20 min. The cells were collected and suspended with a 20 mM phosphate buffer (pH 7.0) to prepare 10% suspension. 0.5% of each Protamex, Flavourzyme, Protease A, Aroase AP-10, Pescalase, Papain or KH-15 was added to the suspension and the suspension was hydrolyzed at 35°C for 6 hrs, heat-treated at 95°C for 10 min, and centrifuged to collect the supernatant. The supernatant was passed through a 10 kDa cut-off membrane (Satocon cassette, Sartorius, Germany) and then freeze-dried to prepare the yeast hydrolysate.

<Experimental embodiment 2> Protein recovery of yeast hydrolysate

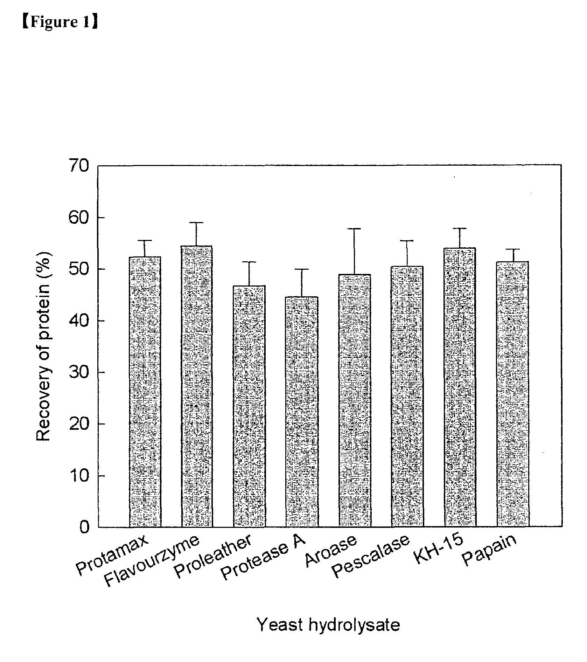

Yeast hydrolysates were prepared using commercial enzymes and protease KH-15 produced by *Bacillus sp.* KH-15 and protein recovery was measured (FIG. 1, Formula 1).

<Formula 1>

$$\text{Protein recovery (\%)} = (\text{Protein after hydrolysis/Protein before hydrolysis}) \times 100$$

The yeast hydrolysate using Protamex or Flavourzyme as a protease exhibited protein recovery of 52.3% and 54.5%, respectively. Protein recoveries of yeast hydrolysates using Proleather, Protease A, and Pescalase, all of which are endo-type proteases, were somewhat low, that is, 46.7%, 44.5%, and 50.4%, respectively, whereas the yeast hydrolysate using KH-15 showed protein recovery of 53.9%, which is similar to protein recoveries of yeast hydrolysates using complex-type (of endo- and exo-type) proteases (Table 2).

[Table 2]

| Enzyme | Protamex | Flavourzyme | Proleather | ProteaseA | Aroase | Pescalase | KH-15 | Papain |
|---|---|---|---|---|---|---|---|---|
| Protein recovery (%) | 52.3±3.2 | 54.5±4.5 | 46.7±4.7 | 44.5±5.4 | 48.9±8.9 | 50.4±5.0 | 53.9±5.0 | 51.3±2.4 |

<Experimental embodiment 3> Inhibitory effect of yeast hydrolysate on fat deposition

Lipolytic effect in adipocytes

3T3-L1 preadiopocytes (ATCC#F8979, Manassas, VA) were cultured in DMEM medium containing 10% fetal bovine serum, 100 unit/mL penicillin, and 100 mg/mL streptomycin in a 5% $CO_2$ incubator. 3 or 4 days later, when cells were confluent, cells were isolated by treating 0.05% trypsin/EDTA. After centrifugation (1,000 rpm, 5 min), cells were collected and adjusted to a density of $3.3 \times 10^3$ cell/cm$^2$ to make suspension. Cell suspension was dispensed into a 12 well plate at 1mL/well and secondary cultured. 3 or 4 days later, when cells were confluent, a differentiation medium (the medium in which 5 $\mu$g/mL insulin, 0.25 $\mu$M dexamethazone, and 0.5mM IBMX are added into DMEM medium) was added to induce cell differentiation.

The medium was replaced with a feeding medium (the medium in which only 5 $\mu$g/mL insulin is contained in DMEM medium) every 2 days and cells were induced to differentiate to adipocytes. After 10 days of the differentiation medium treatment, more than 90% cells were differentiated to adipocytes. Pectin was dissolved to be a concentration of 0.1% in the feeding medium, filtered through a 0.2 $\mu$m filter, and treated to fully differentiated adipocytes.

Glycerol concentration was measured by an enzyme reaction method. 10 $\mu$L of the collected medium was added to 1 mL of a free glycerol reagent preheated to 37°C, and cultured in a 37°C water bath for 5 min. To quantify glycerol, 12.5 $\mu$g and 25 $\mu$g of glycerol standard solution (Sigma) were allowed to react using the same method as with samples and 200 $\mu$L aliquots were taken into a 96-well plate to measure the absorbance.

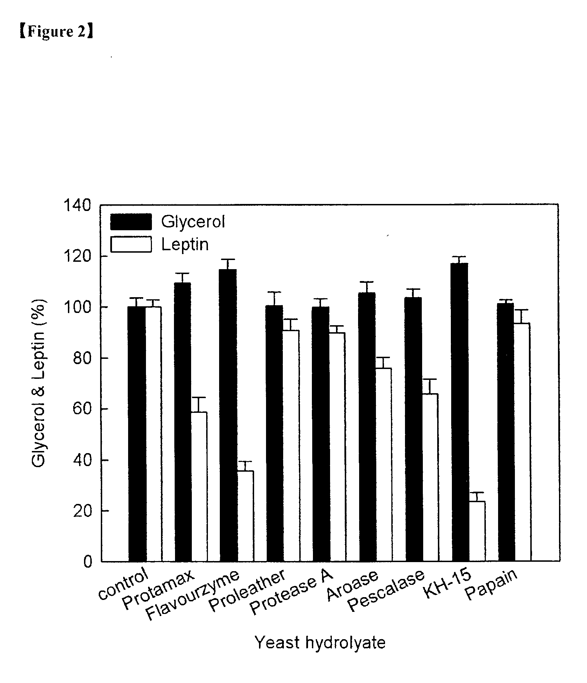

Consequently, the concentrations of glycerol released from lipolysis were increased by 109.3%, 114.5%, and 116.8% in the Protamex-treated group, Flavourzyme-treated group, and KH-15-treated group, compared to control (yeast hydrolysis enzyme-untreated group). However, the Proleather-treated group, Protease A-treated group, Aroase-treated group, Pescalase-treated group, and Papain-treated group did not show great difference from control (FIG. 2).

Inhibitory effect on fat deposition in adipocytes

Leptin, which is a hormone produced by obese gene in adipocytes, is a protein which acts on the hypothalamus to inhibit food intake, increase energy consumption, and control obesity (Caro *et al.,* 1996). Leptin is associated with body fat mass (Considine et al., 1996) and blood leptin level has been known as a biomarker for body fat mass, and recent obesity research has often applied blood leptin level. It has been known that leptin release is increased by increase in fat deposition in adipocytes (Considine *et al.,* 1996). Accordingly, the decrease in leptin level means small deposition of fat.

Leptin level released from adipocytes was measured by Enzyme Linked Immunosolvent Assay (ELISA) method. Rat anti-mouse leptin IgG 2 $\mu$g/mL was cultured in a Maxisorb ELISA plate (Nunc) for one night. 100 $\mu$L of the medium collected from adipocytes was added to the plate washed three times with PBS-T (PBS containing 0.05% Tween 20) buffer and cultured for 1 hr. The plate was washed again with PBS-T three times, and biotinylated rabbit anti-mouse leptin IgG 200 $\mu$g/mL was added thereto. The plate was allowed to stand still at room temperature for 1 hr and washed again with PBS-T three times. Then, extravidin-horse radish peroxidase was incubated at room temperature for 1 hr and washed three times. To measure immunoreactivity, 100 $\mu$L of tetramethylbenzidine dihydrochloride substrate (TMB) was added to each well, allowed to react for 30 min. The reaction was stopped with the addition of 50 $\mu$L of 2M $H_2SO_4$ and the absorbance was measured at 450 nm. Leptin release by pectin was represented as the relative value compared with the control.

When leptin levels in adipocytes by yeast hydrolysates were measured (FIG. 2), treatment with the yeast hydrolysate by KH-15 showed the lowest leptin level, 23.5%. Yeast hydrolysates by Protamex and Flavourzyme showed 58.7% and 35.7% of leptin levels, respectively.

Glycerol concentration was significantly increased and leptin release was greatly decreased by the addition of the yeast hydrolysate by KH-15, suggesting that yeast hydrolysates by KH-15, Protamex, and Flavourzyme have inhibitory effects on fat deposition, independent of lipolysis effects and these two effects are achieved by different mechanisms. In addition, it was concluded that the yeast hydrolysate by KH-15 would have excellent obesity-inhibitory effect, compared to other yeast hydrolysates.

[Table 3]

|  | Control | Protamex | Flavourzyme | Proleather |
|---|---|---|---|---|
| Glycerol (%) | 100.0±3.5 | 109.3±3.9 | 114.5±4.1 | 100.4±5.3 |
| Leptin (%) | 100.0±2.7 | 58.7±5.8 | 35.7±3.7 | 90.6±4.5 |

(continued)

| Protease A | Aroase | Pescalase | KH-15 | Papain |
|---|---|---|---|---|
| 99.8±3.3 | 105.4±4.3 | 103.5±3.4 | 121.8±2.8 | 101.1±1.5 |
| 89.7±2.7 | 75.9±4.3 | 65.8±5.8 | 23.5±3.5 | 93.3±5.3 |

<Experimental embodiment 4> Cytotoxicity of yeast hydrolysate

Cell viability with respect to samples was measured by MTT {3-(4,5-methylthiazol-2-yl)-2,5-diphenylte trazolium bromide} colorimetric assay (Camichael *et al.,* 1978). Cells were dispensed at $1\times10^5$ cell/200 $\mu$L in a 96 well plate, and cultured for 24 hrs, and the medium was removed. In the plate, samples of different concentrations were added to 200 $\mu$L of new DMEM medium, and cultured for 24 hrs. 20 $\mu$L of 2.5 mg/mL MTT solution was added to each well, and incubated for 4 hrs. After incubation, the supernatant was removed and 100 $\mu$L of DMSO was added to each well to dissolve the produced formazan crystals. The absorbance was measured with a microplate reader at 550 nm and the cell viability was calculated as the following <Formula 2>.

<Formula 2>

$$\text{Cell viability (\%)} = \{100 - (\text{absorbance of control} - \text{absorbance of sample treatment})/\text{absorbace of control}\}\times100$$

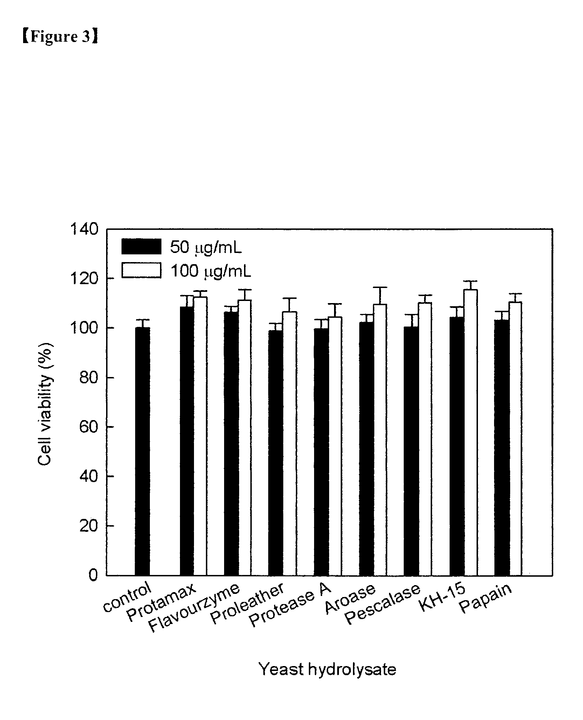

When differentiated 3T3-L1 adipocytes were treated with different concentrations (50, 100 $\mu$g/mL) of yeast hydrolysates for 24 hrs and cytotoxicity was observed, cytotoxicity was not observed within the concentration range. When 100 $\mu$g/mL of yeast hydrolysate was treated, the increase in absorbance at 550 nm was due to the inherent color of sample and there was no cell overproliferation. Accordingly, the concentration to treat adipocytes was established to 100 $\mu$g/mL (FIG. 3). While there were slight differences between yeast hydrolysates, it was found that the yeast hydrolysate at the concentration of 100 $\mu$g/mL did not have cytotoxicity for adipocytes and induced neither proliferation of adipocytes nor obesity.

[Table 4]

| Concentration ($\mu$g/mL) | Control | Protamex | Flavourzyme | Proleather |
|---|---|---|---|---|
| 50 | 100.0±3.3 | 108.4±4.8 | 106.4±2.4 | 98.9±3.1 |
| 100 | | 112.5±2.5 | 111.4±4.1 | 106.5±5.6 |
| **Protease A** | **Aroase** | **Pescalase** | **KH-15** | **Papain** |
| 99.7±3.8 | 102.3±3.2 | 100.5±5.0 | 104.3±4.3 | 103.2±3.5 |
| 104.5±5.4 | 109.6±6.9 | 110.3±3.1 | 115.6±3.5 | 110.5±3.4 |

<Embodiment 3> Activities of yeast KH-15 hydrolysate

<Experimental embodiment 1> Materials

Yeast KH-15 hydrolysate

Physiological activities were measured using the yeast hydrolysate (Eatless) prepared by using KH-15, the protease produced by *Bacillus sp.* KH-15, which showed the most excellent anti-obesity effect in Example 2.

Experimental animals and experimental diets

Male S/D rats weighed about 180 to 185 g were purchased from Daehan Biolink (Umsung, Chungbuk, South Korea). The animals were given *ad libitum* access to the diet (Table 2) and water. Temperature and humidity of a breeding room was kept at 22°C and 40-60% and the dark-light cycle was 12/12 hr.

The rats were divided into two groups (n = 8): the experimental group 1, high-fat diet group (control), and the experimental group 2, high-fat diet group administered orally with the yeast KH-15 hydrolysate (100 mg/kg). Oral administration was done for 24 days.

[Table 5]

| Nutrient | High fat diet (g/100g diet) |
|---|---|
| Casein | 20 |
| Corn starch | 32.3 |
| Sucrose | 10 |
| Lard | 20 |
| Soybean oil | 10 |
| Mineral mixture 1 | 3.5 |
| Vitamin mixture 2 | 1 |
| Cellulose | 3 |
| DL-methionine | 0.2 |
| Total energy(kcal) | 519.2 |
| Percent of calories (per total energy) | |
| Fat | 52 |
| Carbohydrate | 32.6 |
| Protein | 15.4 |
| Diet composition for SD rats | |

<Experimental embodiment 2> Effect of yeast KH-15 hydrolysate on blood lipids of rats

Total cholesterol, HDL-cholesterol, and triglyceride levels in serum were analyzed with a kit reagent by an enzyme method.

After oral administration of yeast KH-15 hydrolysate for 24 days, changes in serum lipids were measured (Table 6). Triglyceride, the causative material for fat deposition, in the high-fat diet group, control, was 96.28 mg/dL and when yeast KH-15 hydrolysate was administered, triglyceride was 80.07 mg/dL. There was a difference between two groups, but it was not a statistically significant difference.

However, the control showed higher total cholesterol level than the yeast KH-15 hydrolysate treatment group. The yeast KH-15 hydrolysate showed about 34% higher HDL-cholesterol level than the high-fat diet group. Therefore, since the yeast KH-15 hydrolysate treatment group showed low total cholesterol level and high HDL-cholesterol level, it can lower the incidence rate of arteriosclerosis.

From the above experimental result, since the yeast KH-15 hydrolysate showed body weight reducing effect and body fat reducing effect, it was confirmed that the yeast KH-15 hydrolysate have inhibitory effect or therapeutic effect on obesity.

[Table 6]

| Group | Triglycerides (mg/dl) | Total cholesterol (mg/dL) | HDL cholesterol (mg/dL) | HTR |
|---|---|---|---|---|
| Control | 96.28 ± 11.31 | 102.45 ± 4.94 | 56.42 ± 2.62 | 0.58 ± 0.04 |

(continued)

| Group | Triglycerides (mg/dl) | Total cholesterol (mg/dL) | HDL cholesterol (mg/dL) | HTR |
|---|---|---|---|---|
| Yeast KH-15 hydrolysate-administered group | 80.07 ± 9.98 | 86.87 ± 5.11* | 75.70 ± 2.79** | 0.85 ± 0.04** |
| Asterisks indicate significant differences compared with control by Students t-tests (*p<0.05, **p<0.01). HTR = HDL cholesterol/Total cholesterol | | | | |

<Experimental embodiment 3> Antioxidant activity of yeast KH-15 hydrolysate

DPPH radical and ABTS radical scavenging activities of yeast KH-15 hydrolysate

DPPH radical scavenging activity was measured using the method of Cheung et al (2003). 0.4 mL of 0.2 mM DPPH solution in which DPPH was dissolved in ethanol was allowed to react with 0.1 mL of sample for 10 min in the dark and the absorbance was measured at 520 nm.

ABTS radical scavenging activity was measured using the method of Re et al (1999). 2.45 mM potassium persulfate was added to 7 mM ABTS, and allowed to stand still in the dark at room temperature for 12-16 hrs. The ABTS radical solution was diluted with distilled water to an absorbance of 1.4-1.5 at 414 nm. 250 $\mu$L of the diluted ABTS radical solution was added to 12.5 $\mu$L of sample and allowed to react for 60 min in the dark and the absorbance was measured at 414 nm. DPPH and ABTS radical scavenging activities (%) were calculated as the following <Formula 3>. <Formula 3>

$$\text{Radical scavenging activity (\%)} = (1 - A_{\text{sample}}/A_{\text{control}}) \times 100$$

$A_{\text{sample}}$: with sample, $A_{\text{control}}$: without sample

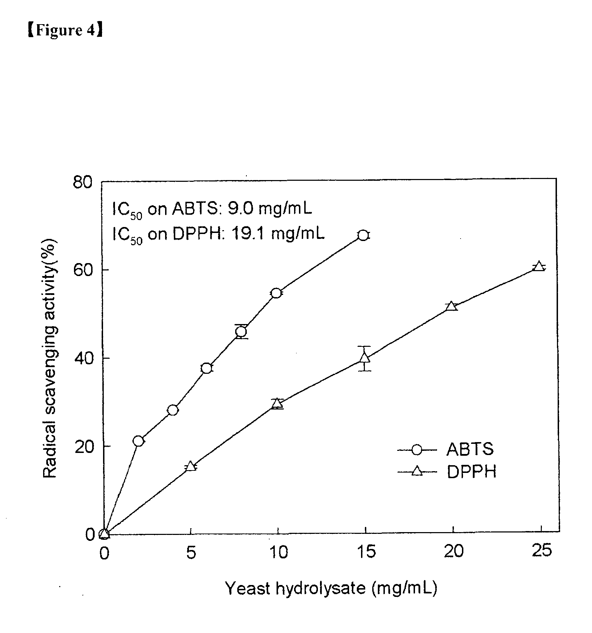

DPPH radical scavenging activity and ABTS radical scavenging activity of the yeast KH-15 hydrolysate was increased in a concentration-dependent way as shown in FIG. 4. The $IC_{50}$ values required to scavenge 50% of radicals were 19.1 mg/mL and 9.0 mg/mL and it was confirmed that the yeast KH-15 hydrolysate has relatively high level radical scavenging activities. Therefore, with the addition of the yeast KH-15 hydrolysate to feeds, antioxidant activity may be expected.

Effects of yeast KH-15 hydrolysate on reduced glutathione and lipid peroxides

Oxygen free radicals are highly toxic materials produced in cells which survive using oxygen and damage cellular DNAs and lipids and proteins in cell membrane. Therefore, there are antioxidant enzymes such as superoxide dismutase (SOD), catalase, glutathione peroxidase, and the like, and various nonenzymatic antioxidant materials such as glutathione, uric acid, and the like in cells, and they protect various cellular structures from oxygen free radical-induced oxidative stress. SOD transforms oxygen free radicals to $H_2O_2$ and $O_2$, and catalase and glutathione peroxidase scavenge the produced $H_2O_2$. Glutathione peroxidase scavenges $H_2O_2$ while oxidizing reduced glutathione (GSH) to oxidized glutathione (GSSG), and GSSG is reduced back to GSH by glutathione reductase, and glutathione exist within the human body in two forms, GSH and GSSG, which are kept in balance. If toxicity or oxidative damage occurs in the cell, GSSG slowly increases and this results in the GSH/GSSG imbalance, and the role as a protective mechanism disappears. GSH has been regarded as an important material which protects oxygen free radicals-induced cellular damage and plays a detoxification role within the cell. Therefore, cells in a healthy individual can be protected sufficiently by a protective mechanism scavenging oxygen free radicals which can cause oxidative cellular damages (De Haan et al., 1995).

The reduced glutathione level in blood was measured using the method of Tietz (1969). The same amount of 5% sulfosalicylic acid was added to the blood plasma, and centrifuged at 4°C, 2,000 × g for 10 min. 800 $\mu$L of 0.3mM $Na_2HPO_4$ and 100 $\mu$L of the solution in which 5,5'-dithiobis 2-nitrobenzoic acid (DTNB) was mixed to 0.04% with 0.1% sodium citrate were added to 100 $\mu$L of the supernatant and 5 min later, the absorbance was measured at 412 nm. Quantification was done using the reduced glutathione as a standard material.

Lipid peroxide level in blood was measured using the method of Quintanilha et al (1982). 200 $\mu$L of 10% trichloroacetic acid was added to 100 $\mu$L of blood plasma and allowed to stand still at room temperature for 15 min, and centrifuged at 4°C, 2,200 × g for 15 min. 200 $\mu$L of 0.67% thiobarbituric acid was added to and mixed with 200 $\mu$L of the supernatant, and allowed to react in a 100°C constant temperature water bath for 10 min, and cooled. The absorbance was measured at 532 nm, and quantification was done using malondialdehyde (MDA) as a standard material.

The result of blood and hepatic GSH levels in SD rats administered orally with the yeast KH-15 hydrolysate was as shown in Table 7. The higher the level of MDA (malondialdehyde) which is the oxidation product of lipids in blood, the larger oxidative stress is. MDA levels in control and the yeast KH-15 hydrolysate treatment group were 12.0 $\mu$mol/g and 9.0 $\mu$mol/g, respectively. It was confirmed that fewer oxidation product MDA was produced by oral administration of the yeast KH-15 hydrolysate. In addition, reduced GSH (glutathione sulfate) levels responsible for antioxidant activities in blood and liver were 437.4 $\mu$mol/L and 6.7 mmol/L, high in the yeast KH-15 hydrolysate oral administration group, compared to control. The increase in GSH responsible for antioxidation and decrease in the oxidation product MDA were observed by oral administration of the yeast KH-15 hydrolysate. This means that the yeast KH-15 hydrolysate has not only anti-obesity effect, but also the effect to scavenge radicals, the causative material of various diseases.

[Table 7]

| Group | MDA | GSH | |
|---|---|---|---|
| | Liver ($\mu$mol/g) | Serum ($\mu$mol/mL) | Liver (mmol/g) |
| Control | 12.0$\pm$3.7[b] | 253.3$\pm$52.1[b] | 4.6$\pm$0.9[b] |
| Yeast KH-15 hydrolysate-administered group | 9.0$\pm$1.7[a] | 437.4$\pm$223.7[a] | 6.7$\pm$1.2[a] |
| Hepatic and blood MDA and GSH levels by administration of yeast KH-15 hydrolysate | | | |

<Experimental embodiment 3> Effects of yeast KH-15 hydrolysate on fat deposition and obesity

Body weight gain and dietary intake

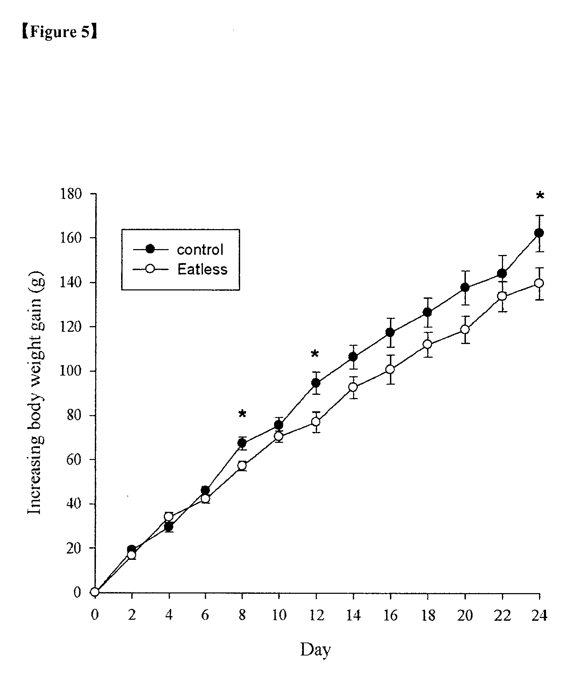

Body weight gain of rats was measured from the high-fat diet group and the yeast KH-15 oral administration group (100 mg/kg, administration for 24 days). Since 12th day of oral administration, there was a significant difference in body weight gain between the high-fat diet group and the yeast KH-15 hydrolysate treatment group, and it was confirmed that body weight gain in the yeast KH-15 hydrolysate treatment group was small (FIG. 5).

Daily average body weight gain was measured (Table 8). The body weight gain in the yeast KH-15 hydrolysate oral administration group was 5.63 g, smaller than that of control. Food intake in the yeast KH-15 hydrolysate oral administration group was slightly smaller than that of control, but it was not significant. However, since the body weight gain in the yeast KH-15 hydrolysate oral administration group was small, it was confirmed that the dietary efficiency was decreased.

After oral administration of the yeast KH-15 hydrolysate for 24 days, the body weight gain was significantly small, compared to control and there was an obvious difference in daily average body weight. Thus, the yeast KH-15 hydrolysate is expected to have excellent obesity-preventive effect.

[Table 8]

| Group | Dietary intake (g/day) | Body weight gain (g/day) | Dietary efficiency |
|---|---|---|---|
| Control | 17.36$\pm$0.46 | 7.28$\pm$0.76 | 0.42$\pm$0.04 |
| Yeast KH-15 hydrolysate-administered group | 17.21$\pm$0.60 | 5.63$\pm$0.63 | 0.33$\pm$0.04 |
| Values are the means$\pm$SD for 8 rats. Dietary efficiency (Body weight gain (g/day)/Dietary intake (g/day)) | | | |

Body fat reducing effect

Organ weight changes of each experimental group, there was no significant difference in organ weight changes of liver, spleen, and kidney. However, while the epididymal fat pad and the perirenal fat pad of control were 5.38 g and 3.78 g, the epididymal fat pad and the perirenal fat pad of the yeast KH-15 hydrolysate oral administration group were 4.38 g and 3.05 g.

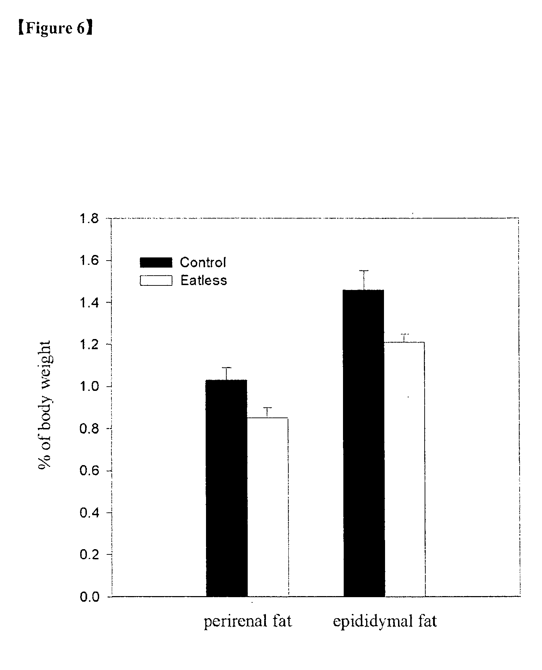

The ratio of fat weight to body weight was measured (FIG. 6). The ratio of the epididymal fat pad and the perirenal fat pad in the high-fat diet group were 1.46% and 1.03%, whereas the ratio of epididymal fat pad and the perirenal fat pad in the yeast KH-15 hydrolysate oral administration group were 1.21% and 0.85%. Thus, the oral administration of the yeast KH-15 hydrolysate decreased the epididymal fat pad and the perirenal fat pad by 17.1% and 17.5%, compared to control.

Accordingly, it was confirmed that the administration of the yeast KH-15 hydrolysate decreased the deposition of fat, the causative material of obesity, significantly.

<Embodiment 4> Activities of yeast KH-15 hydrolysate

<Experimental embodiment 1> Materials

Yeast KH-15 hydrolysate

Physiological activities were measured using the yeast hydrolysate (Eatless) prepared by using KH-15, the protease produced by *Bacillus sp.* KH-15, which showed the most excellent anti-obesity effect from the above results.

Experimental animals and experimental diets

Dogs (beagles) (2-5 years old) were given *ad libitum* access to a standard feed (Purina) and water. The breeding room was maintained under conditions of temperature 24°C, humidity 40-60%, and lighting of a 12-hour light/dark cycle. 100 mg/kg of capsules containing the yeast hydrolysate were orally administered. Beagles were divided into three groups (control, experimental group 1, and experimental group 2) (5 beagles/group). The control was fed a standard feed. The experimental group 1 was fed a standard feed with oral administration of the yeast KH-15 hydrolysate (100 mg/kg) for 30 days. The experimental group 2 was fed a standard feed for the first 10 days, a standard feed and the yeast KH-15 hydrolysate between day 11th and day 20th, and a standard feed between day 21st and day 30th, respectively. Feeding pattern of each group was as follows (Table 9). Initial preference of dogs to the yeast KH-15 hydrolysate was fairly good and the yeast KH-15 hydrolysate is seemed to be suitable for a feed additive.

[Table 9]

| Group | Day 1st - 10th | Day 11th - 20th | Day 21st - 30th |
|---|---|---|---|
| Control | Standard feed | Standard feed | Standard feed |
| Experimental group 1 (Eatless-A) | Standard feed + yeast KH-15 hydrolysate | Standard feed + yeast KH-15 hydrolysate | Standard feed + yeast KH-15 hydrolysate |
| Experimental group 2 (Eatless-B) | Standard feed | Standard feed + yeast KH-15 hydrolysate | Standard feed |
| Feeding pattern of experimental groups | | | |

<Experimental embodiment 2> Effects of yeast KH-15 hydrolysate on fat deposition and obesity

Body weight gain

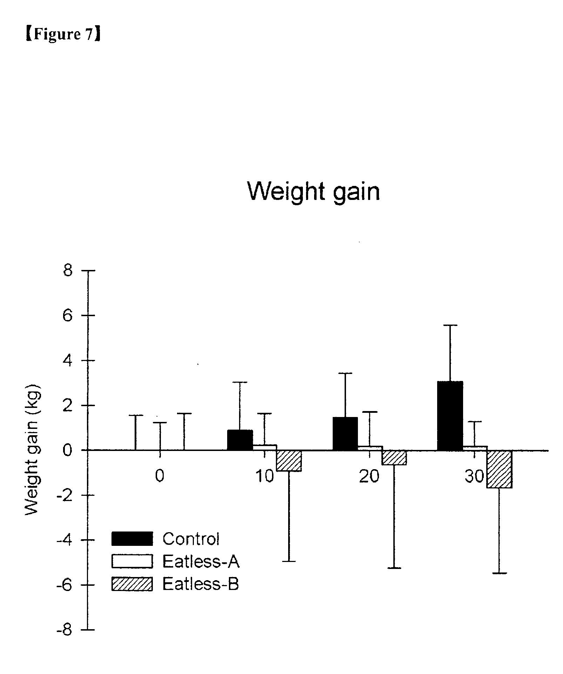

A standard feed and the yeast KH-15 hydrolysate were orally administered respectively for 30 days, and body weight gain was measured before the experiment, 10, 20, and 30 days after beginning the experiment (FIG. 7). Body weight gain in control was increased with the passage of time, and body weight gains on 10th day, 20th day, and 30th day were 0.88 kg, 1.46 kg, and 3.06 kg, respectively. Meanwhile, the experimental group 1 (Eatless-A) showed 0.2 kg of body weight gain after 30 days and the experimental group 2 (Eatless-B) showed 1.64 kg of body weight reduction.

Abdominal circumference

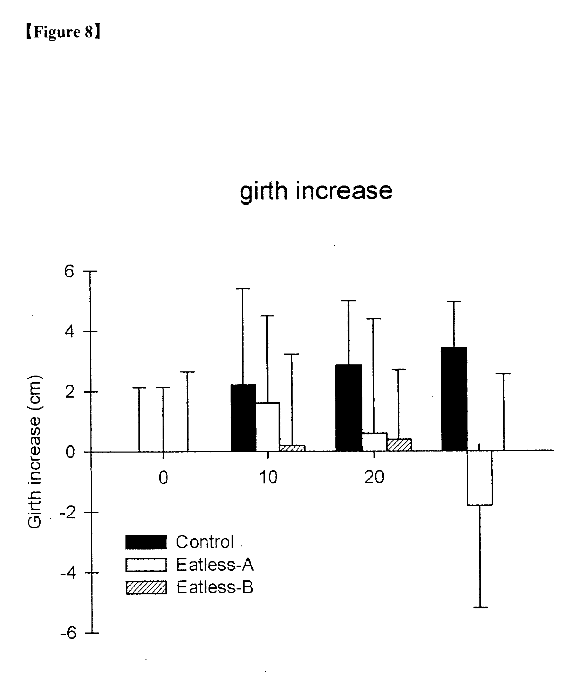

Abdominal circumferences were measured (FIG. 8). The control group fed only a standard feed showed body weight gain and increases in abdominal circumferences were 2.2 cm on 10th day, 2.86 cm on 20th day, and 3.43 cm on 30th day. However, the abdominal circumference in the experimental group 1 was decreased by 1.8 cm on 30th day and the abdominal circumference in the experimental group 2 did not change on 30th day.

From the above results, there was a slight difference depending on the administration method, but it was confirmed that the oral administration of the yeast KH-15 hydrolysate can inhibit body weight gain and induce body weight reduction.

<Experimental embodiment 3> Measurement of blood biomarkers after administration of yeast KH-15 hydrolysate

As stated above, it was confirmed that the oral administration of the yeast KH-15 hydrolysate has inhibitory effect on body weight gain, or rather, body weight reducing effect. This may be result from toxicity of the yeast KH-15 hydrolysate. Accordingly, blood samples were collected and several components in blood which are measured during physical examination of pet dogs were measured (Table 10 and Table 11).

Total cholesterol, HDL-cholesterol, triglyceride levels in serum were analyzed with kit reagents by enzymatic methods. Red blood cells, white blood cells, and hemoglobin level in blood were examined (Table 10). It was confirmed that all biomarkers were within the normal range in control and the yeast KH-15 hydrolysate oral administration groups, the experimental group 1, and the experimental group 2.

[Table 10]

| Biomarker | Control | Experimental group 1 | Experimental group 2 | Normal range |
|---|---|---|---|---|
| Red blood cells ($10^{12}$/L) | 6.0±0.1 | 5.8±0.3 | 6.3±0.2 | 4.7-7.1 |
| White blood cells ($10^9$/L) | 7.2±0.4 | 8.3±0.4 | 7.9±0.4 | 5-12 |
| Hemoglobin (g/100 mL) | 13.0±0.7 | 13.5±0.45 | 12.7±0.6 | 9-15 |
| Red blood cell, white blood cell, and hemoglobin levels in dogs' blood | | | | |

Cholesterol, albumin, ALT (alanine aminotransferase), and so on in blood plasma were measured (Table 11). When biomarkers in blood plasma were measured, cholesterol, albumin, and protein levels were also within normal range in each group. Especially, the biomarker of liver injury, ALT value was also within normal range in each group. This is the result confirming indirectly that the body weight reducing effect shown in the experimental group 2 by oral administration of the yeast KH-15 hydrolysate was not caused by the toxicity of the yeast KH-15 hydrolysate. Since several biomarkers are within normal range, compared to other values in Table 7 and Table 8, it seemed that such body weight reduction was caused by the anti-obesity activity of the yeast KH-15 hydrolysate.

[Table 11]

| Biomarker | Control | Experimental group 1 | Experimental group 2 | Normal range |
|---|---|---|---|---|
| Total cholesterol (mmol/L) | 3.68±0.8 | 3.41±0.78 | 3.54±0.43 | 3.5-7.25 |
| Total protein (g/L) | 54.6±2.1 | 56.8±1.8 | 53.8±1.2 | 50-62 |
| Albumin (g/L) | 32.1±0.9 | 31.5±1.4 | 30.8±2.1 | 27-38 |
| Globulin (g/L) | 22.3±2.1 | 25.7±0.9 | 24.6±1.6 | 17-30 |
| Alanine aminotransferase(U/L) | 28.0±4.5 | 34.5±2.7 | 36.7±3.5 | 0-77 |
| Alkaline phosphatase (U/L) | 90.4±8.9 | 99.5±5.6 | 100.2±6.2 | 0-174 |
| Plasma biochemical markers in dogs | | | | |

<Experimental embodiment 4> Antioxidant activity of yeast KH-15 hydrolysate

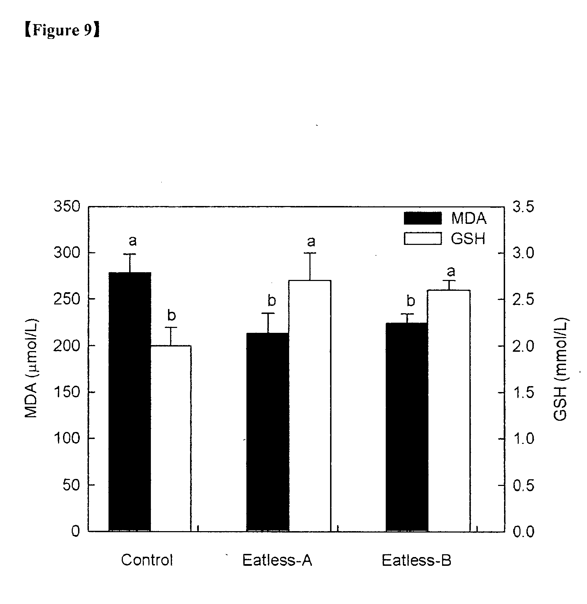

The biomarkers of antioxidant activities, MDA and GSH levels in blood were measured. The oxidation product, MDA (malondialdehyde) level was 278.4 μmol/L, which was high in the control group. Meanwhile, the yeast KH-15 hydrolysate administration groups, the experimental group 1 and group 2 showed 213.6 μmol/L and 224.4 μmol/L of MDA and those values were small. In addition, the antioxidant material GSH level was 2.0 mmol/L in the control group, whereas the experimental group 1 and group 2 showed slightly high contents, 2.7 mmol/L and 2.6 mmol/L of GSH, respectively (FIG. 9).

[Sequence Listing Free Text]

<SEQ 10 NO:1>

MNQKAMIVIA  AGSMLFGGAG  VYAGINLLEM  DKPQTAAVPA

TAQADSERDK AMDKIEKAYE

LISNEYVEKV  DREKLLEGAI  QGMLSTLNDP  YSVYMDKQTA

KGGSDSLDSS FEGIGAEVGM

EDGKIIIVSP  FKKSPAEKAG  LKPLSTIISI  NGESMAGKDL

NHAVLKIRGK KGSSVSMKIQ

RPGTKKQLSF  RIKRAEIPLE  TVFASEKKVQ  GHSVGYIAIS

TFSEHTAEDF AKALRELEKK

EIEGLVIDVR  GNPGGYLQSV  EEILKHFVTK  DQPYIQIAER

NGDKKRYFST LTHKKAYPVN

VITDKGSASA  SEILAGALKE  AGHYDVVGDT  SFGKGTVQQA

VPMGDGSNIK LTLYKWLTPN

GNWIHKKGIE  PTIAIKQPDY  FSAGPLQLKE  PLKVDMNNED

VKHAQVLLKG LSFDPGREDG

YFSKDMKKAV  MAFQDQNKLN  KTGVIDTRTA  ETLNQQIEKK

KSDEKNDLQL QTALKASFVN

<110> Neo Cremar Co.,Ltd

<120> Yeast hydrolysate which has obesity treatment and antioxidative function

<130> DAP09229PCT

<150> KR10-2010-0039717
<151> 2010-04-28

<160> 1

<170> KopatentIn 1.71

<210> 1
<211> 480
<212> PRT
<213> Artificial Sequence

<220>
<223> protease produced by Bacilus sp. KH-15 isolated from soybean paste

<400> 1

```
Met Asn Gln Lys Ala Met Ile Val Ile Ala Ala Gly Ser Met Leu Phe
 1            5                 10            Ser         15

Gly Gly Ala Gly Val Tyr Ala Gly Ile Asn Leu Leu Glu Met Asp Lys
            20                25                30

Pro Gln Thr Ala Ala Val Pro Ala Thr Ala Gln Ala Asp Ser Glu Arg
            35                40                45

Asp Lys Ala Met Asp Lys Ile Glu Lys Ala Tyr Glu Leu Ile Ser Asn
        50                55                60

Glu Tyr Val Glu Lys Val Asp Arg Glu Lys Leu Leu Glu Gly Ala Ile
65                70                75                80

Gln Gly Met Leu Ser Thr Leu Asn Asp Pro Tyr Ser Val Tyr Met Asp
            85                90                95

Lys Gln Thr Ala Lys Gly Gly Ser Asp Ser Leu Asp Ser Ser Phe Glu
            100               105               110

Gly Ile Gly Ala Glu Val Gly Met Glu Asp Gly Lys Ile Ile Ile Val
            115               120               125

Ser Pro Phe Lys Lys Ser Pro Ala Glu Lys Ala Gly Leu Lys Pro Leu
    130               135               140

Ser Thr Ile Ile Ser Ile Asn Gly Glu Ser Met Ala Gly Lys Asp Leu
145               150               155               160

Asn His Ala Val Leu Lys Ile Arg Gly Lys Lys Gly Ser Ser Val Ser
            165               170               175

Met Lys Ile Gln Arg Pro Gly Thr Lys Lys Gln Leu Ser Phe Arg Ile
        180               185               190

Lys Arg Ala Glu Ile Pro Leu Glu Thr Val Phe Ala Ser Glu Lys Lys
        195               200               205

Val Gln Gly His Ser Val Gly Tyr Ile Ala Ile Ser Thr Phe Ser Glu
```

```
           210                    215                    220

    His Thr Ala Glu Asp Phe Ala Lys Ala Leu Arg Glu Leu Glu Lys Lys
    225                 230             235                 240

    Glu Ile Glu Gly Leu Val Ile Asp Val Arg Gly Asn Pro Gly Gly Tyr
                    245             250                 255

    Leu Gln Ser Val Glu Glu Ile Leu Lys His Phe Val Thr Lys Asp Gln
                    260             265                 270

    Pro Tyr Ile Gln Ile Ala Glu Arg Asn Gly Asp Lys Lys Arg Tyr Phe
                275             280             285

    Ser Thr Leu Thr His Lys Lys Ala Tyr Pro Val Asn Val Ile Thr Asp
        290             295             300

    Lys Gly Ser Ala Ser Ala Ser Glu Ile Leu Ala Gly Ala Leu Lys Glu
    305             310             315             320

    Ala Gly His Tyr Asp Val Val Gly Asp Thr Ser Phe Gly Lys Gly Thr
                325             330             335

    Val Gln Gln Ala Val Pro Met Gly Asp Gly Ser Asn Ile Lys Leu Thr
                340             345             350

    Leu Tyr Lys Trp Leu Thr Pro Asn Gly Asn Trp Ile His Lys Lys Gly
            355             360             365

    Ile Glu Pro Thr Ile Ala Ile Lys Gln Pro Asp Tyr Phe Ser Ala Gly
        370             375             380

    Pro Leu Gln Leu Lys Glu Pro Leu Lys Val Asp Met Asn Asn Glu Asp
    385             390             395             400

    Val Lys His Ala Gln Val Leu Leu Lys Gly Leu Ser Phe Asp Pro Gly
                405             410             415

    Arg Glu Asp Gly Tyr Phe Ser Lys Asp Met Lys Lys Ala Val Met Ala
                420             425             430

    Phe Gln Asp Gln Asn Lys Leu Asn Lys Thr Gly Val Ile Asp Thr Arg
                435             440             445

    Thr Ala Glu Thr Leu Asn Gln Gln Ile Glu Lys Lys Lys Ser Asp Glu
        450             455             460

    Lys Asn Asp Leu Gln Leu Gln Thr Ala Leu Lys Ala Ser Phe Val Asn
    465             470             475             480
```

<110> Neo Cremar Co.,Ltd

<120> Yeast hydrolysate which has obesity treatment and antioxidative function

<130> DAP09229PCT

<150> KR10-2010-0039717
<151> 2010-04-28

<160> 1

<170> KopatentIn 1.71


<210> 1
<211> 480
<212> PRT
<213> Artificial Sequence


<220>
<223> protease produced by Bacilus sp. KH-15 isolated from soybean paste


<400> 1


```
Met Asn Gln Lys Ala Met Ile Val Ile Ala Ala Gly Ser Met Leu Phe
 1               5                  10                  15

Gly Gly Ala Gly Val Tyr Ala Gly Ile Asn Leu Leu Glu Met Asp Lys
                20                  25                  30

Pro Gln Thr Ala Ala Val Pro Ala Thr Ala Gln Ala Asp Ser Glu Arg
                35                  40                  45

Asp Lys Ala Met Asp Lys Ile Glu Lys Ala Tyr Glu Leu Ile Ser Asn
        50                  55                  60

Glu Tyr Val Glu Lys Val Asp Arg Glu Lys Leu Leu Glu Gly Ala Ile
65                  70                  75                  80

Gln Gly Met Leu Ser Thr Leu Asn Asp Pro Tyr Ser Val Tyr Met Asp
                85                  90                  95

Lys Gln Thr Ala Lys Gly Gly Ser Asp Ser Leu Asp Ser Ser Phe Glu
                100                 105                 110

Gly Ile Gly Ala Glu Val Gly Met Glu Asp Gly Lys Ile Ile Ile Val
                115                 120                 125

Ser Pro Phe Lys Lys Ser Pro Ala Glu Lys Ala Gly Leu Lys Pro Leu
        130                 135                 140

Ser Thr Ile Ile Ser Ile Asn Gly Glu Ser Met Ala Gly Lys Asp Leu
145                 150                 155                 160

Asn His Ala Val Leu Lys Ile Arg Gly Lys Lys Gly Ser Ser Val Ser
                165                 170                 175

Met Lys Ile Gln Arg Pro Gly Thr Lys Lys Gln Leu Ser Phe Arg Ile
                180                 185                 190

Lys Arg Ala Glu Ile Pro Leu Glu Thr Val Phe Ala Ser Glu Lys Lys
                195                 200                 205

Val Gln Gly His Ser Val Gly Tyr Ile Ala Ile Ser Thr Phe Ser Glu
```

```
        210                     215                     220
    His Thr Ala Glu Asp Phe Ala Lys Ala Leu Arg Glu Leu Glu Lys Lys
    225                 230                 235                 240

    Glu Ile Glu Gly Leu Val Ile Asp Val Arg Gly Asn Pro Gly Gly Tyr
                    245                 250                 255

    Leu Gln Ser Val Glu Glu Ile Leu Lys His Phe Val Thr Lys Asp Gln
                260                 265                 270

    Pro Tyr Ile Gln Ile Ala Glu Arg Asn Gly Asp Lys Lys Arg Tyr Phe
            275                 280                 285

    Ser Thr Leu Thr His Lys Lys Ala Tyr Pro Val Asn Val Ile Thr Asp
    290                 295                 300

    Lys Gly Ser Ala Ser Ala Ser Glu Ile Leu Ala Gly Ala Leu Lys Glu
    305                 310                 315                 320

    Ala Gly His Tyr Asp Val Val Gly Asp Thr Ser Phe Gly Lys Gly Thr
                325                 330                 335

    Val Gln Gln Ala Val Pro Met Gly Asp Gly Ser Asn Ile Lys Leu Thr
                340                 345                 350

    Leu Tyr Lys Trp Leu Thr Pro Asn Gly Asn Trp Ile His Lys Lys Gly
            355                 360                 365

    Ile Glu Pro Thr Ile Ala Ile Lys Gln Pro Asp Tyr Phe Ser Ala Gly
        370                 375                 380

    Pro Leu Gln Leu Lys Glu Pro Leu Lys Val Asp Met Asn Asn Glu Asp
    385                 390                 395                 400

    Val Lys His Ala Gln Val Leu Leu Lys Gly Leu Ser Phe Asp Pro Gly
                405                 410                 415

    Arg Glu Asp Gly Tyr Phe Ser Lys Asp Met Lys Lys Ala Val Met Ala
                420                 425                 430

    Phe Gln Asp Gln Asn Lys Leu Asn Lys Thr Gly Val Ile Asp Thr Arg
                435                 440                 445

    Thr Ala Glu Thr Leu Asn Gln Gln Ile Glu Lys Lys Lys Ser Asp Glu
            450                 455                 460

    Lys Asn Asp Leu Gln Leu Gln Thr Ala Leu Lys Ala Ser Phe Val Asn
    465                 470                 475                 480
```

**Claims**

1. A composition having weight reducing activity through inhibition of fat deposition and antioxidant activity comprising a yeast hydrolysate obtained by proteolyzing *Saccharomyces cerevisiae* with protease KH-15 consisting of the amino acid sequence of SEQ ID NO:1.

2. A composition comprising a yeast hydrolysate obtained by proteolyzing *Saccharomyces cerevisiae* with protease KH-15 consisting of the amino acid sequence of SEQ ID NO:1, for the use in preventing or treating arteriosclerosis, visceral obesity, abdominal obesity, hyperlipidemia, fatty liver, or obesity.

**Patentansprüche**

1. Zusammensetzung, die durch Hemmung der Fettablagerung und antioxidative Aktivität gewichtsreduzierende Aktivität aufweist, umfassend ein Hefe-Hydrolysat, erhalten durch die Proteolyse von *Saccharomyces cerevisiae* mit der Protease KH-15, die aus der Aminosäuresequenz von SEQ ID NO:1 besteht.

2. Zusammensetzung, die ein Hefe-Hydrolysat umfasst, erhalten durch die Proteolyse von *Saccharomyces cerevisiae* mit der Protease KH-15, die aus der Aminosäuresequenz von SEQ ID NO: 1 besteht, für die Verwendung bei der Vorbeugung oder Behandlung von Arteriosklerose, viszeraler Adipositas, abdominaler Adipositas, Hyperlipidämie, Fettleber oder Adipositas.

**Revendications**

1. Composition ayant une activité de réduction du poids par une inhibition du dépôt de graisse et une activité antioxydante, comprenant un hydrolysat de levure obtenu par une protéolyse de *Saccharomyces cerevisiae* avec la protéase KH-15 consistant en la séquence d'acides aminés de SEQ ID NO: 1.

2. Composition comprenant un hydrolysat de levure obtenu par une protéolyse de *Saccharomyces cerevisiae* avec la protéase KH-15 consistant en la séquence d'acides aminés de SEQ ID NO: 1, destinée à être utilisée dans la prévention ou le traitement de l'artériosclérose, l'obésité viscérale, l'obésité abdominale, l'hyperlipidémie, la stéatose hépatique ou l'obésité.

【Figure 1】

【Figure 2】

【Figure 3】

【Figure 4】

IC$_{50}$ on ABTS: 9.0 mg/mL
IC$_{50}$ on DPPH: 19.1 mg/mL

Radical scavenging activity(%)

Yeast hydrolysate (mg/mL)

—○— ABTS
—△— DPPH

【Figure 5】

【Figure 6】

【Figure 7】

Weight gain

【Figure 8】

girth increase

【Figure 9】

**EP 2 564 860 B1**

**Patent documents cited in the description**

- KR 1020100039717